Europäisches Patentamt

European Patent Office

Office européen des brevets

(11) Veröffentlichungsnummer: **0 377 453**
**A1**

## EUROPÄISCHE PATENTANMELDUNG

(21) Anmeldenummer: **90100094.3**

(22) Anmeldetag: **03.01.90**

(51) Int. Cl.5: **A61M 25/01**

(30) Priorität: **04.01.89 DE 8900077 U**

(43) Veröffentlichungstag der Anmeldung:
**11.07.90 Patentblatt 90/28**

(84) Benannte Vertragsstaaten:
**AT BE DE DK ES FR GB IT NL SE**

(71) Anmelder: **Baker, Susan**
**41 Rusham Park Avenue**
**Egham, Surrey(GB)**

(72) Erfinder: **Baker, Susan**
**41 Rusham Park Avenue**
**Egham, Surrey(GB)**

(74) Vertreter: **Patentanwälte Dr. Solf & Zapf**
**Zeppelinstrasse 53**
**D-8000 München 80(DE)**

(54) **Führungsdraht.**

(57) Bei einem Führungsdraht (1), z. B. zum Einführen eines Katheters, mit einem insbesondere durch eine J-förmig gekrümmte Spitze (2) gebildeten vorderen Einführende und einem sich daran anschließenden Führungsstrang (3) mit Führungsabschnitten (7, 8, 9) unterschiedlicher Flexibilität, wird der Führungsstrang (3) durch eine bis zum hinteren Ende (4) reichende einstückige, einteilige Drahtseele (5) gebildet, die abschnittweise eine unterschiedliche Steifigkeit aufweist.

FIG.1

EP 0 377 453 A1

## Führungsdraht

Die Erfindung betrifft einen Führungsdraht, z.B. zum Einführen eines Katheters, mit einem flexiblen Einführende und Führungsabschnitten unterschiedlicher Flexibilität.

Ein solcher Führungsdraht wird z.B. im DE-GM 86 08 051 beschrieben. Bei diesem bekannten Führungsdraht soll sich an das flexible, J-förmig gebogene Einführende ein relativ steifer Führungsabschnitt anschließen, dem wiederum ein flexiblerer Abschnitt folgen soll. An den dem Einführabschnitt abgewandten Ende des Führungsdrahtes soll schließlich noch ein relativ steifer Aufsetzabschnitt angeordnet sein. Die relativ steifen Abschnitte sollen aus Volldraht bestehen und die flexiblen Abschnitte schraubenförmig gewendelten Draht aufweisen. Es können aber auch mehradrige verdrillte oder geflochtene Litzen für die flexiblen Abschnitte vorgesehen sein. Der erste relativ steife Abschnitt ragt mit einem sich verjüngenden Endabschnitt in den flexiblen schraubenförmig gewickelten Einführabschnitt hinein.

Dieser bekannte Führungsdraht ist kompliziert aufgebaut. Die einzelnen Abschnitte sind miteinander verschweißt. Die Herstellung ist daher sehr aufwendig.

Bei dem in der DE-OS 35 16 052 beschriebenen Führungsdraht sind eine weichflexible, J-förmig gekrümmte Spitze, ein relativ steifer Schaft und ein flexibel-biegbarer Strangkörper aus Metall vorgesehen. Die weich-flexible Spitze ist aus einem mit sehr geringer Steigung schraubenförmig enggewickelten dünnen Draht aufgebaut, in dessen Innern ein exzentrischer Fangdraht in Form eines flachen Bandes verläuft, dessen äußeres Ende in einen Spitzenkörper eingeschweißt ist, der als halbkugelförmig verrundeter Schweißpunkt ausge bildet ist und den Kopf des gewickelten Drahtes bildet, mit dem er ebenfalls verschweißt ist. Die weichflexible Spitze ist J-förmig gekrümmt und in ihrem geraden Teil befindet sich ein gegen den Krümmungsteil verjüngter Stiftkörper, dessen stärkeres Ende in den relativ steifen Schaft hineinragt und sowohl mit diesem als auch mit dem Draht der Spitze fest verlötet ist. Die unterschiedlich flexiblen Teile sind miteinander verlötet. Für diesen bekannten Führungsdraht trifft ebenfalls zu, daß er sehr kompliziert aufgebaut und die Herstellung teuer ist.

Die Verwirklichung unterschiedlich flexibler Abschnitte bei Führungsdrähten wird in noch komplizierterer Form in der US-PS 2 221 138 beschrieben. Durch zur Spitze auslaufende Verjüngungen wird die Flexibilität - ähnlich wie bei den oben beschriebenen bekannten Ausführungsformen der Spitze des Führungsdrahtes - erhöht.

Ein anderer Führungsmandrin bzw. Führungsdraht nach dem DE-GM 83 28 562 besteht aus einer Drahtseele, die von einem Mantel aus schraubenlinienförmig gewickeltem Draht umgeben ist und die am proximalen Mandrinende gegenüber dem Mantel zurückgesetzt ist. Der Mantel besteht aus mehreren, die Drahtseele schraubenlinienförmig umgebenden, flach geneigt zur Mandrinachse verlaufenden und nebeneinander liegenden Einzeldrähten, wobei sich zwischen dem Mantel und der Drahtseele eine Kunststoffzwischenschicht befindet. Der Raum zwischen dem proximalen Ende des Mantels und dem proximalen Ende der zurückgesetzten Drahtseele ist mit Kunststoff gefüllt. Zudem kann vorgesehen sein, daß der Mantel mit einer Kunststoffaußenschicht umgeben ist, die am proximalen Mandrinende eine Abschlußkappe bildet. Die Drahtseele kann aus Federstahlmaterial bestehen.

Dieser bekannte Führungsmandrin, der nach Art einer Litze hergestellt ist, soll eine hohe Flexibilität haben und eine Verformung auch bei sehr kleinen Biegeradien ermöglichen. Die besondere Ausbildung des Mantels am proximalen Ende der Drahtseele ergibt keine abrupte Stabilitäts- und Flexibilitätsänderung; vielmehr verändern sich diese Größen kontinuierlich, so daß ein Abknicken in diesem Bereich vermieden werden kann. Die unterschiedliche Flexibilität des Führungsmandrins auf der Lauflänge wird durch entsprechende Materialwahl und Dimensionierung der Drahtseele und der dem Mantel bildenden Einzeldrähte gewährleistet.

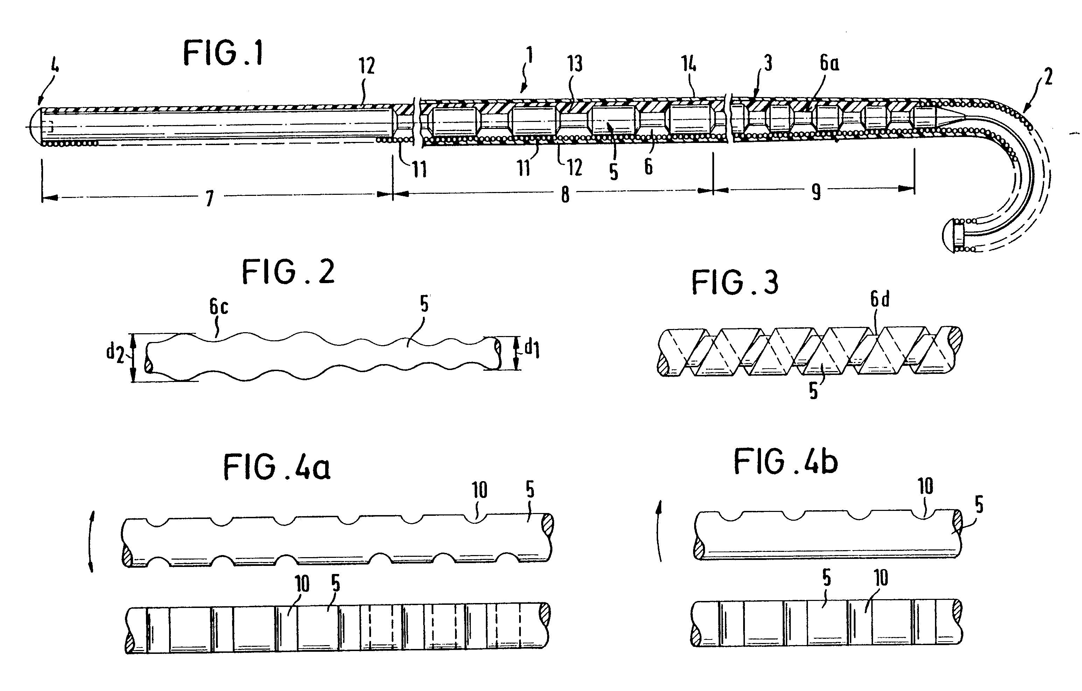

Im Übergang zwischen der zurückgesetzten Drahtseele und der flexiblen Spitze des Mantels wird eine ausreichende Stabilität gegen Umknicken des proximalen Mandrinendes dadurch erreicht, daß sich die Drahtseele zum proximalen Ende hin verjüngt und außerdem dadurch, daß die Einzeldrähte des Mantels flach geneigt zur Mandrinachse verlaufen. Die Herstellung des sich verjüngenden proximalen Bereichs erfolgt u.a. gemäß Fig. 4 über ein Zwischenprodukt, bei dem von einem Kerndraht ausgegangen wird, der sich z.B. alle 30 cm auf einer Länge von 3 bis 5 cm bis zu einer Einziehung verjügt. Diese Verjüngung kann dadurch erreicht werden, daß der Draht alle 30 cm auf eine Temperatur von etwa 400 bis 800°C erwärmt und durch Strecken verjüngt wird. Nach anschließendem Abschrecken sollen die Eigenschaften des hierfür verwendeten Federstahldrahtes erhalten bleiben. Das Zwischenprodukt wird ummantelt und gegebenenfalls noch mit einer Außenschicht versehen, anschließend an der Einziehung durchtrennt und die Trennstelle mit einer Abschlußkappe versehen. Die Verjüngung der Drahtseele kann auch durch Rundhämmern bewirkt werden.

Die Gewährleistung der unterschiedlichen Flexibilität der Lauflänge des bekannten Führungsmandrins ist ebenso wie die beiden anderen beschriebenen Führungsdrähte nur mit erheblichem Aufwand sicherzustellen.

Aufgabe der Erfindung ist, mit einfachen Mitteln einen Führungsdraht zu schaffen, der auf der Lauflänge unterschiedliche Flexibilität aufweist, wobei die Flexibilität mit einfachen Mitteln erzeugt wird.

Diese Aufgabe wird durch die kennzeichnenden Merkmale der Ansprüche 1 oder 25 gelöst. Vorteilhafte Weiterbildungen der Erfindung werden in den davon abhängigen Unteransprüchen gekennzeichnet.

Anhand der Zeichnung wird die Erfindung im folgenden beispielhaft näher erläutert. Es zeigen:

Fig. 1 einen Schnitt durch den Mantel eines Führungsdrahts,

Fig. 2 bis 5 und 7 Varianten der Drahtseele des Führungsdrahts,

Fig. 6 eine andere Ausführungsform des Führungsdrahtes.

Der Führungsdraht 1 gemäß Fig. 1 weist eine weich-flexible J-förmig gekrümmte Spitze 2 bekannten Aufbaus auf. An die Spitze 2 schließt sich nach hinten der eigentliche Führungsstrang 3 an mit einer vorzugsweise bis zum Ende 4 reichenden einstückigen, einteiligen Drahtseele 5. Wesentlich ist, daß die Drahtseele 5 abschnittsweise eine unterschiedliche Biegsamkeit bzw. ein unterschiedliches Biegemoment bzw. unterschiedliche Steifigkeit aufweist.

Diese unterschiedliche Steifigkeit wird zweckmäßigerweise durch Querschnittsverjüngungen 6 erreicht, die jeweils oder von Abschnitt zu Abschnitt einen unterschiedlichen Durchmesser aufweisen können, woraus die unterschiedliche Steifigkeit resultiert. Im dargestellten Beispiel gemäß Fig. 1 gibt es einen Abschnitt 7 aus Vollmaterial der Drahtseele 5. Es folgt ein Abschnitt 8 mit gleichförmigen, ringförmigen Querschnitts verjüngungen 6 in Form von Ringnuten 6a oder Hinterdrehungen im gleichen Abstand voneinander. Daran schließt sich ein Abschnitt 9 an mit kürzeren ringförmigen Hinterdrehungen oder Ringnuten 6a gleichen oder sich insbesondere zur Spitze 2 hin stufenförmig verringernden Durchmessers, woraus eine Zone größerer Steifigkeit resultiert.

Nach der Erfindung können von Abschnitt zu Abschnitt die Länge, die Tiefe und/oder der Abstand voneinander der Querschnittsverjüngungen 6 variiert sein zur Gewährleistung unterschiedlicher Steifigkeit.

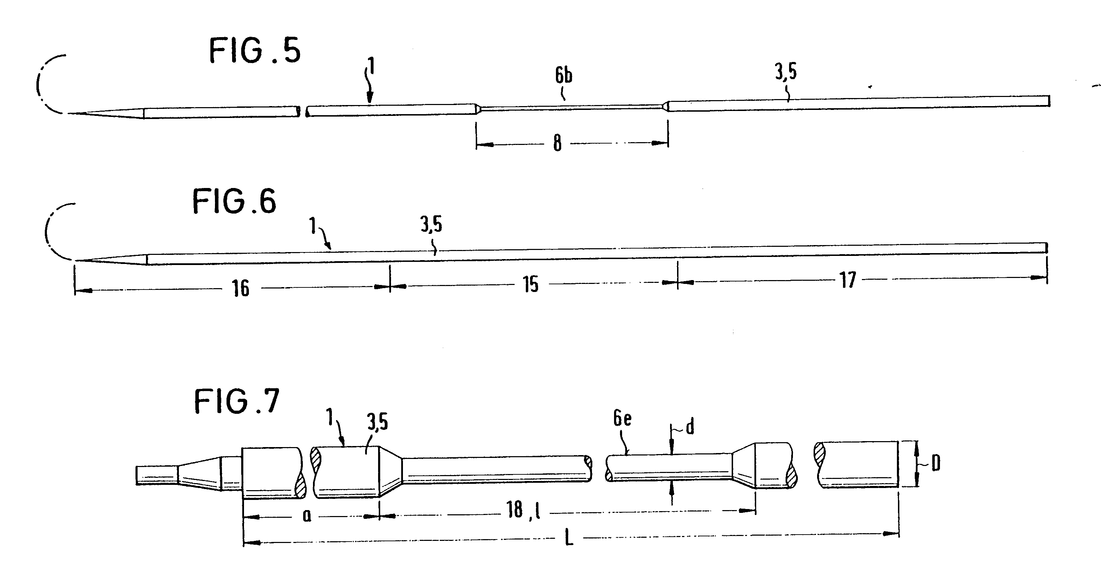

Im Beispiel nach Fig. 5 ist nur eine Hinterdrehung oder Ringnut 6b im Abschnitt 8 vorgesehen, die aber eine erhebliche Länge von etwa 100 bis 800 mm haben kann bei einer Gesamtlänge des Führungsdrahtes 1 von etwa 800 mm bis 1200 mm. Die Hinterdrehungen oder Ringnuten 6a, 6b brauchen nicht - im Längsschnitt betrachtet - die trapezförmige Form gemäß Fig. 1 und 5 besitzen. Die Fig. 2 zeigt eine wellenförmige Hinterdrehung oder wellenförmige Ringnuten 6c, und die Fig. 3 eine gewindeförmige Hinterdrehung oder Ringnut 6c, die ebenfalls unterschiedliche Steifigkeiten bewirken.

Bei den Beispielen nach Fig. 4a und 4b sind die Querschnittsverjüngungen 6 durch Ausnehmungen 10 in der Drahtseele 5 gebildet, die sich quer zur Längsachse der Drahtseele 5 erstrekken. Die Ausnehmungen 10 können sich gegenüberliegend oder axial versetzt (Fig. 4a, linke und rechte Hälfte) und auch diametral zueinander versetzt angeordnet sein, oder sie sind nur einseitig entlang einer Mantellinie der Drahtseele 5 eingebracht (Fig. 4b).

Bei der Ausführungsform gemäß Fig. 6 weist der aus Volldraht bestehende Führungsstrang 3 bzw. Drahtseele 5 wenigstens einen Abschnitt unterschiedlicher Steifigkeit, hier einen mittleren Abschnitt 15 geringerer Steifigkeit auf, der eine geringere oder größere Härte als der wenigstens eine harte oder weiche übrige Abschnitte oder Abschnitte 16, 17 aufweist oder durch Weichglühen eines härteren Abschnitts 16, 17 gebildet ist.

Bei dieser Ausführungsform der Erfindung ist somit vorgesehen, daß die Abschnitte 15, 16, 17 der Drahtseele 5 zwar einteilig und einstückig aus dem gleichen Material bestehen, wobei die Abschnitte jedoch unterschiedliche E-Modulen aufweisen. Dies wird erfindungsgemäß z.B. durch Härten oder Weichglühen des Materials in bestimmten Abschnitten erreicht. Diese Variante der Erfindung kann aber auch vorteilhafterweise mit den oben anhand der Fig. 1 bis 5 beschriebenen Ausführungsformen kombiniert sein.

Beim Ausführungsbeispiel gemäß Fig. 7 weist der Führungsstrang 3 bzw. die Drahtseele 5 einen mittleren Abschnitt 18 geringerer Steifigkeit auf, der durch eine Querschnittsverjüngung 6 längerer Abmessung in Form einer im Querschnitt trapezförmigen Ringnut 6e gebildet ist. Der Abstand a der Ringnut 6e vom mit 19 bezeichneten Anschlußstück, an das die Spitze 2 angelötet ist (nicht dargestellt), beträgt etwa 300 mm. Bei einer Länge L des Führungsstranges 3 von etwa 950 mm beträgt die Länge l der Ringnut 6e etwa 550 mm, wobei sie sich bis zum oder kurz vor das hintere Ende erstrecken kann. Beim vorliegenden Ausführungsbeispiel beträgt der Durchmesser D des Vollquerschnitts des Führungsstrangs 3 etwa 0,85 mm, wobei die Ringnut 6e auf einen Durchmesser d von etwa 0,4 mm verjüngt ist.

Die Querschnittsverjüngungen 6 lassen sich durch spanabhebende Bearbeitung, Prägung, Walzen oder Ätzen herstellen. Hierdurch ist nicht nur eine preiswerte Herstellung möglich, sondern es wird der Fläche der Querschnittsverjüngung 6 auch eine vorteilhafte Raumform bzw. Oberflächenbeschaffenheit verliehen, wobei eine Feinbearbeitung zu bevorzugen ist, um eine Kerbwirkung zu vermeiden und eine lange Lebensdauer der Drahtseele 5 zu erhalten. Hierzu eignet sich insbesondere das Einarbeiten der Querschnittsverjüngungen 6 durch Ätzen, wobei für das Ätzen eine gerundete oder trapezförmige Querschnittsform der Querschnittsverjüngung 6 vorzuziehen ist.

Der erfindungsgemäße Führungsdraht 1 kann ohne Umhüllung, wie in Fig. 5 abgebildet, verwendet werden. Es ist aber bevorzugt, die Drahtseele 5 mit einem schraubenlinienförmig enggewickelten Draht 11 oder mit einer Kunststoffbeschichtung 12 oder mit beiden zu versehen, wobei in letzterem Fall zweckmäßigerweise der Draht 11 unter der Kunststoffbeschichtung 12 angeordnet ist. Zweckmäßig ist ferner, zunächst eine die Querschnittsverjüngungen 6 ausgleichende Kunststoffbeschichtung 13 vorzusehen und darauf den Draht 11 und/oder die Kunststoffbeschichtung 14 anzuordnen. Diese Varianten sind in Fig. 1 abgebildet.

Mit der Erfindung ist ein völlig anderer Weg beschritten worden, unterschiedliche Steifigkeiten zu gewährleisten. Dieser Weg führt zu einer derartigen Vereinfachung der Herstellung des Führungsdrahts, daß alle bisher bekannten Führungsdrahttypen nicht mehr konkurrieren können. Die Vorteile sind nicht nur wirtschaftlicher Art, sondern betreffen auch die Eigenschaften der Abschnitte, die bei insbesondere den Übergangsbereichen zwischen dem einen und dem anderen Abschnitt mit Problemen behaftet waren. Die Eigenschaften der Drahtseele in den Übergangsbereichen waren meist undefinierbar.

## Ansprüche

1. Führungsdraht, z.B. zum Einführen eines Katheters, mit einem insbesondere durch eine J-förmig gekrümmte Spitze (2) gebildeten vorderen Einführende und einem sich daran anschließenden Führungsstrang (3) mit Führungsabschnitten unterschiedlicher Flexibilität, dadurch **gekennzeichnet,** daß der Führungsstrang (3) durch eine bis zum hinteren Ende (4) reichende einstückige, einteilige Drahtseele (5) gebildet ist, die abschnittweise eine unterschiedliche Steifigkeit aufweist.

2. Führungsdraht nach Anspruch 1, dadurch **gekennzeichnet,** daß die Drahtseele (5) in dem wenigstens einen Abschnitt (8, 9, 15, 18) geringerer Steifigkeit eine oder mehrere Querschnittsverjüngungen (6, 6a, 6c, 6d, 6e) aufweist.

3. Führungsdraht nach Anspruch 2, dadurch **gekennzeichnet,** daß die Querschnittsverjüngungen (6) dieses Abschnitts (9) oder von Abschnitt (8) zu Abschnitt (9) eine unterschiedliche Tiefe und/oder Länge aufweisen.

4. Führungsdraht nach einem oder mehreren der Ansprüche 1 bis 3, dadurch **gekennzeichnet,** daß die Querschnittsverjüngungen (6) dieses Abschnitts oder von Abschnitt zu Abschnitt unterschiedliche Abstände voneinander aufweisen.

5. Führungsdraht nach einem der Ansprüche 3 oder 4, dadurch **gekennzeichnet,** daß die Tiefe, Länge und/oder die Abstände kontinuierlich ab- oder zunehmen.

6. Führungsdraht nach einem oder mehreren der Ansprüche bis 5, dadurch **gekennzeichnet,** daß die Querschnittsverjüngung oder -verjüngungen (6, 6b) im Längsschnitt betrachtet eine trapezförmige Form aufweist oder aufweisen.

7. Führungsdraht nach einem oder mehreren der Ansprüche 1 bis 5, dadurch **gekennzeichnet,** daß die Querschnittsverjüngung oder -verjüngungen (6c, 10) im Längsschnitt betrachtet eine kreisabschnittförmige Form aufweist oder aufweisen.

8. Führungsdraht nach einem oder mehreren der Ansprüche 1 bis 7, dadurch **gekennzeichnet,** daß die Querschnittsverjüngungen (6c) wellenförmig ausgebildet sind.

9. Führungsdraht nach einem oder mehreren der Ansprüche 1 bis 8, dadurch **gekennzeichnet,** daß die Querschnittsverjüngung oder -verjüngungen (6a, 6c, 6e) jeweils durch eine allseitige Taillierung, Ringausnehmung oder Hinterdrehung gebildet ist oder sind.

10. Führungsdraht nach Anspruch 9, dadurch **gekennzeichnet,** daß sein Querschnitt (d) im Bereich der Taillierung oder Ringausnehmung (6a, 6c, 6e) rund ist.

11. Führungsdraht nach einem oder mehreren der Ansprüche 1 bis 8, dadurch **gekennzeichnet,** daß die Querschnittsverjüngungen (6d) gewindeförmig ausgebildet sind.

12. Führungsdraht nach einem oder mehreren der Ansprüche 1 bis 11, dadurch **gekennzeichnet,** daß die Querschnittsverjüngung oder - verjüngungen in der Drahtseele (5) durch wenigstens eine sich quer zur Längs achse der Drahtseele (5) erstreckende Ausnehmung (10) gebildet ist oder sind, die auf einer oder auf beiden Seiten der Drahtseele (5) angeordnet ist oder sind.

13. Führungsdraht nach Anspruch 12, dadurch **gekennzeichnet,** daß die beiderseits der Drahtseele (5) angeordneten Ausnehmungen (10) diametral oder in Längsrichtung versetzt zueinander angeordnet sind.

14. Führungsdraht nach einem oder mehreren der vorhergehenden Ansprüche, dadurch **gekennzeichnet,** daß die Querschnittsverjüngung bzw. -verjüngungen (6) oder die Ausnehmung bzw. Ausnehmungen (10) symmetrisch geformt und/oder angeordnet sind.

15. Führungsdraht nach einem oder mehreren der vorhergehenden Ansprüche, dadurch **gekennzeichnet,** daß die Drahtseele (5) nur einen Abschnitt geringerer Steifigkeit mit vorzugsweise einer Querschnittsverjüngung (6b) in ihrem mittleren oder hinteren Bereich aufweist.

16. Führungsdraht nach Anspruch 15, dadurch **gekennzeichnet,** daß bei einer Länge (L) der Drahtseele (5) von etwa 800 bis 1200 mm der Abschnitt (18) etwa 100 bis 800 mm, vorzugsweise 120 bis 150 mm oder 550 mm lang ist und einen Abstand (a) von vorzugsweise etwa 300 mm vom Anschlußende (19) für die Spitze (2) aufweist.

17. Führungsdraht nach Anspruch 16, dadurch **gekennzeichnet,** daß bei einem Durchmesser der Drahtseele (5) von etwa 0,8 bis 0,9 mm der Durchmesser (d) der Querschnittsverjüngung (6e) etwa 0,4 mm beträgt.

18. Führungsdraht nach einem oder mehreren der Ansprüche 1 bis 17, dadurch **gekennzeichnet,** daß die Fläche der wenigstens einen Querschnittsverjüngung (6) oder Ausnehmung (10) spanabhebend bearbeitet ist.

19. Führungsdraht nach einem oder mehreren der Ansprüche 1 bis 17, dadurch **gekennzeichnet,** daß die Fläche der wenigstens einen Querschnittsverjüngung (6) oder Ausnehmung (10) geprägt oder gewalzt ist.

20. Führungsdraht nach wenigstens einem oder mehreren der Ansprüche 1 bis 17, dadurch **gekennzeichnet,** daß die Fläche der wenigstens einen Querschnittsverjüngung (6) oder Ausnehmung (10) geätzt ist.

21. Führungsdraht nach einem oder mehreren der Ansprüche 1 bis 20, dadurch **gekennzeichnet,** daß die Drahtseele (5) mit einem schraubenlinienförmig enggewickelten Draht (11) umgeben ist.

22. Führungsdraht nach einem oder mehreren der Ansprüche 1 bis 21, dadurch **gekennzeichnet,** daß die Drahtseele (5) eine Kunststoffbeschichtung (12) aufweist.

23. Führungsdraht nach einem oder mehreren der Ansprüche 1 bis 22, dadurch **gekennzeichnet,** daß die Drahtseele (5) zunächst mit einem enggewickelten Draht (11) umgeben ist und auf der Drahtwicklung einer Kunststoffbeschichtung (12) vorgesehen ist.

24. Führungsdraht nach einem oder mehreren der Ansprüche 1 bis 23, dadurch **gekennzeichnet,** daß eine die Querschnittsverjüngung oder -verjüngungen (6, 10) ausgleichende Kunststoffbeschichtung (13) vorgesehen ist.

25. Führungsdraht nach dem Oberbegriff des Anspruchs 1 oder nach einem oder mehreren der Ansprüche 1 bis 24, dadurch **gekennzeichnet,** daß die Abschnitte (15, 16, 17) unterschiedlicher Steifigkeit durch Härten und/oder Weichglühen gebildet sind.

26. Führungsdraht nach einem oder mehreren der Ansprüche 1 bis 25, dadurch **gekennzeichnet,** daß die Querschnittsverjüngung oder -verjüngungen mit gehärteten und/oder weichgeglühten Abschnitten oder Zonen kombiniert ist oder sind.

FIG.1

FIG.2

FIG.3

FIG.4a

FIG.4b

EP 0 377 453 A1

FIG.5

FIG.6

FIG.7

EP 0 377 453 A1

| | EINSCHLÄGIGE DOKUMENTE | | EP 90100094.3 |

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| X | <u>WO - A1 - 88/04 940</u> (TERUMO) | 1,21, 22,25 | A 61 M 25/01 |
| X,P | * Zusammenfassung * & EP-A1-0 340 304 (TERUMO) * Zusammenfassung; Seite 3, Zeilen 10-24,32-35; Seite 4, Zeilen 11-32; Seite 6, Zeilen 2-31; Seite 7, Zeilen 25-33; Seite 8, Zeilen 1-35; Patentansprüche 1,5,9,11,16,17 * -- | | |
| X | <u>US - A - 4 721 117</u> (C.E.MAR et.al.) * Fig; Spalte 1, Zeilen 47-51; Spalte 1, Zeile 64 - Spalte 2, Zeile 14 Spalte 2, Zeilen 38-44 * -- | 1,18, 21 | |
| D,A | <u>DE - U1 - 8 328 562</u> (P.MÄRZ et.al.) * Fig. 1; Seite 10, Zeilen 1-16 * -- | 1,22 | |
| | | | **RECHERCHIERTE SACHGEBIETE (Int. Cl.⁵)** |
| D,A | <u>DE - A1 - 3 516 052</u> (B.BRAUN MELS.) * Gesamt * ---- | 1,21 | A 61 M 25/00 |

Der vorliegende Recherchenbericht wurde für alle Patentansprüche erstellt.

| Recherchenort | Abschlußdatum der Recherche | Prüfer |
|---|---|---|
| WIEN | 19-04-1990 | LUDWIG |